# FASCICULE DE BREVET EUROPEEN

(11) **EP 0 446 092 B1**
(45) Date de publication et mention de la délivrance du brevet: **26.07.1995**
(21) Numéro de dépôt: 91400464.3
(22) Date de dépôt: 20.02.1991
(51) Int. Cl.: A61B 17/58

(54) **Dispositif de liaison transversale rigide entre deux tiges d'ostéosynthèse rachidienne**
Starres transversales Bindeglied zwischen zwei Wirbelsäulenstützstäben
Device for rigid transverse connection of two spinal osteosynthesis rods

(30) Priorité: 08.03.1990 FR 9002970
(43) Date de publication de la demande: 11.09.1991
(73) Titulaire: SOCIETE DE FABRICATION DE MATERIEL ORTHOPEDIQUE SOFAMOR, 75016 Paris (FR)
(72) Inventeur: Cotrel, Yves, Paul, 75015 Paris (FR)
(74) Mandataire: Martin, Jean-Paul

(56) Documents cités:
- EP-A- 0 348 272
- DE-C- 653 799
- FR-A- 2 244 446
- US-A- 2 187 642

## Description

La présente invention a pour objet un élément de fixation pour dispositif de fixation transverse destiné à assurer une liaison rigide entre deux tiges d'un système d'ostéosynthèse rachidienne, ainsi que le dispositif de fixation transverse équipé de ces éléments.

On sait que dans l'ostéosynthèse rachidienne selon la technique COTREL-DUBOUSSET, on utilise un dispositif de liaison transverse (appelé DTT en abrégé) composé d'un crochet fixe muni d'une tige filetée, et de trois crochets libres permettant la prise de rapprochement ou d'écartement de deux tiges moletées, grâce à des écrous et des vis(FR-A2244446).

Ce système, nécessaire pour garantir la stabilité du matériel d'ostéosynthèse en forme de cadre, présente les inconvénients suivants :
- le temps nécessaire à sa mise en place est relativement long,
- sa mise en place est assez difficile,
- sa rigidité en torsion et en flexion est relativement faible.

L'invention a donc pour but de remédier à ces inconvénients et a pour objet un élément de fixation conforme au préambule de la revendication 1.

Suivant l'invention, l'élément de fixation est conforme à la partie caractérisante de la revendication 1.

Suivant une particularité de l'invention, les deux lames sont recourbées suivant un rayon de courbure correspondant à celui des tiges, et ainsi adaptées pour envelopper celles-ci.

Selon une autre caractéristique, la portée est plane afin de pouvoir prendre appui sur une face plane d'une barre de section rectangulaire, les deux lames s'étendant parallèlement l'une à l'autre de part et d'autre de ladite portée.

Le dispositif de fixation transverse également visé par l'invention comprend en combinaison une barre rigide de longueur appropriée, et une paire d'éléments de fixation de la barre sur les tiges tels que mentionnés ci-dessus, pouvant être fixés de manière réglable en une position choisie sur la barre.

Ce matériel est simplifié par rapport au matériel antérieurement utilisé, donc plus rapide et plus aisé à mettre en place par le chirurgien. En outre il présente une rigidité en torsion et en flexion beaucoup plus élevée, grâce à la barre dont la section est élevée et constante sur toute sa longueur.

L'invention sera maintenant décrite en référence aux dessins annexés qui en illustrent une forme de réalisation à titre d'exemple non limitatif.

La figure 1 est une vue en perspective éclatée d'une forme de réalisation du dispositif de fixation transverse selon l'invention.

La figure 2 est une vue en perspective à échelle agrandie par rapport, à la Fig.1, de l'un des éléments de fixation du dispositif.

La figure 3 est une vue en perspective éclatée sous un angle différent des Fig.1 et 2, de l'un des crochet du dispositif, ainsi que de son bouchon de blocage sur la tige enveloppée par ce crochet.

La figure 4 est une vue en perspective du dispositif des Fig.1 à 3 assemblé, vu sous les crochets, du côté des tiges du dispositif.

La figure 5 est une vue en élévation partielle de la barre du dispositif des Fig.1 à 4 chevauchée par un crochet.

La figure 6 est une vue en perspective du dispositif de liaison transverse selon les Fig.1 à 5, mis en place sur un segment rachidien.

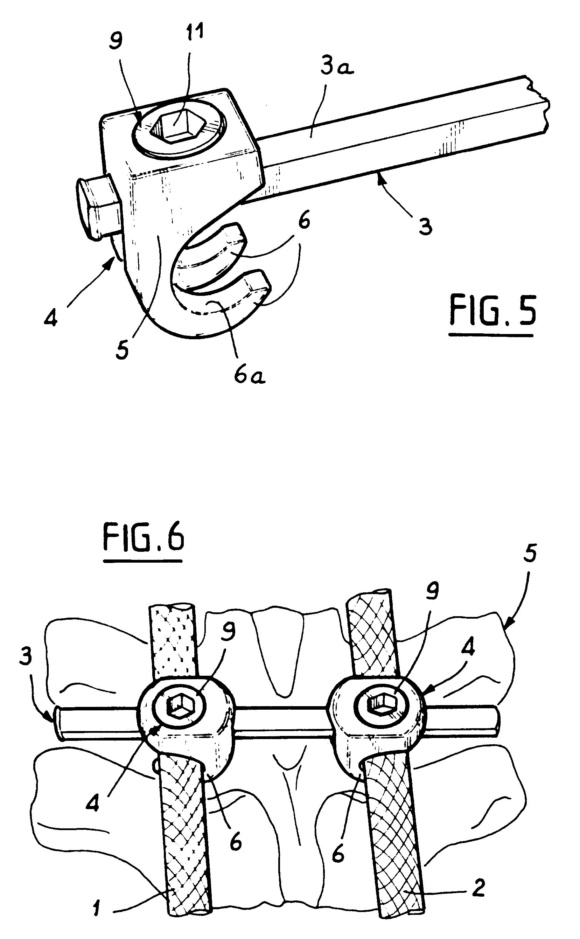

On voit aux dessins un dispositif de fixation transverse destiné à assurer une liaison transversale rigide entre deux tiges 1 et 2 d'un système d'ostéosynthèse rachidienne devant être mis en place sur un segment rachidien S, partiellement visible à la Fig.6.

Ce dispositif comprend, d'une part une barre rigide 3, coupée à une longueur appropriée, légèrement supérieure à l'écartement entre les deux tiges 1 et 2, et une paire d'éléments de fixation 4, pouvant être fixés de manière réglable en une position choisie sur la barre 3. Cette dernière a une section adaptée, par exemple rectangulaire.

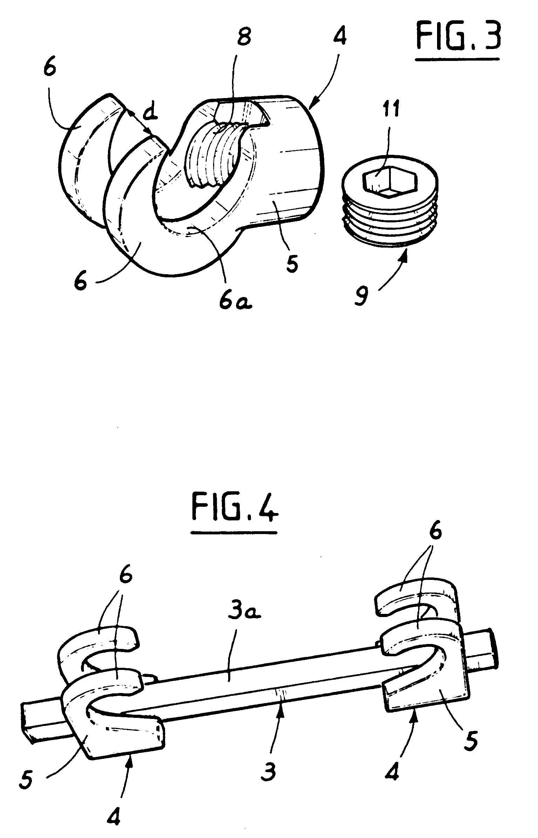

Chaque élément 4 est constitué par un crochet adapté pour pouvoir coiffer la barre 3 de manière coulissante. A cet effet chaque crochet 4 comprend un corps 5 et deux lames 6 s'étendant parallèlement l'une à l'autre, séparées par un intervalle d (Fig.3) dont la largeur correspond à celle de l'une des faces 3a de la barre 3 de section rectangulaire, afin de pouvoir chevaucher la barre en s'étendant de part et d'autre de celle-ci. Les deux lames 6 sont recourbées suivant un rayon de courbure correspondant à celui des tiges moletées 1 et 2, afin de pouvoir envelopper celles-ci dans leurs surfaces d'appui 6a, par exemple sur un peu plus d'une demi-circonférence.

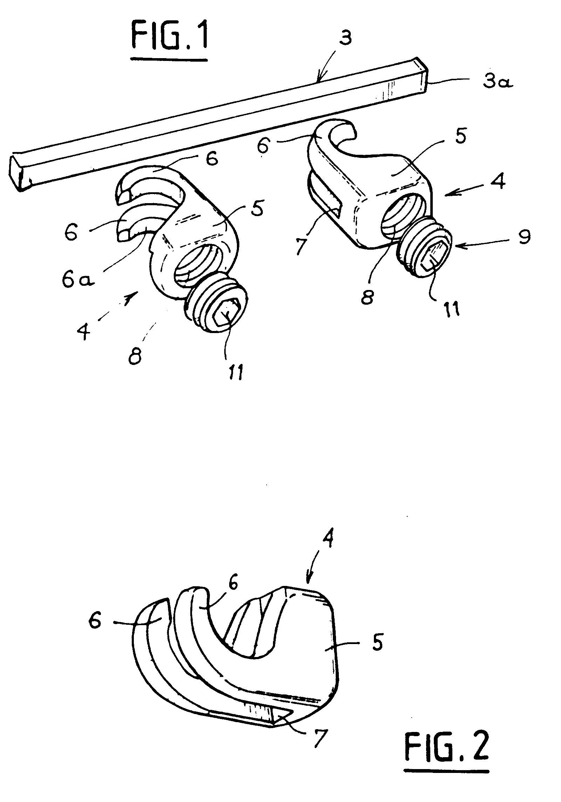

Entre la base des deux lames 6 et sur le corps 5, est formée une portée 7 d'appui d'une face 3a de la barre 3, cette portée 7 constituant le fond du logement de la barre dans le crochet 4. La portée 7 s'étend d'une extrémité à l'autre du corps 5, et dans sa zone centrale elle est interrompue par un trou taraudé 8 (Fig.1 et 3) agencé dans le corps 5. Un bouchon fileté 9 est adapté pour pouvoir être vissé dans le trou taraudé correspondant 8, de façon en fin de vissage à venir s'appuyer sur la face 3a de la barre 3 placée en butée sur la portée 7, et à bloquer la barre 3 par serrage entre le bouchon 9 et la tige 1 (ou 2). La face d'appui du bouchon 9 sur la barre 3 est plane ou éventuellement munie de moyens d'accrochage tels qu'une pointe. Dans chaque bouchon 9 est ménagé un orifice profilé 11, par exemple un six pans adapté pour recevoir un outil non représenté de vissage du bouchon 9.

Pour mettre en place le dispositif qui vient d'être décrit, le chirurgien pose la barre 3 sur les tiges 2, puis installe les deux crochets 4 en les faisant enfourcher la barre 3 pour envelopper les tiges 1, 2. Selon le montage choisi, les crochets 4 sont placés soit en écartement des tiges 1 et 2 (Fig.6), soit en rapprochement de ces tiges. Dans la position en écartement, les lames 6 sont orientées dans des directions opposées l'une à l'autre, leur base se trouvant entre les tiges 1 et 2. Par contre dans la position en rapprochement, les crochets 4 sont placés de manière que leurs lames 5 soient orientées l'une vers l'autre, leur base étant située à l'extérieur des tiges 1 et 2.

La fixation de la barre 3 sur les tiges 1 et 2 s'effectue donc après que le chirurgien ait écarté ou rapproché les deux crochets 4, l'entraxe étant conservé en serrant les vis ou bouchons 9 de blocage des crochets 4 dans leur position choisie sur la barre 3. Cette dernière est alors fixe par rapport aux deux tiges 1 et 2, et assure la cohésion ou la stabilité du système d'ostéosynthèse, avec une forte rigidité en torsion et en flexion.

L'invention n'est pas limitée au mode de réalisation décrit et peut comporter des variantes d'exécution. Ainsi éventuellement la section de la barre 3 pourrait être autre que rectangulaire, et les bouchons ou vis 9 de blocage des crochets 4 peuvent être remplacés par tout moyen équivalent.

## Revendications

1. Elément de fixation (4) pour dispositif de fixation transverse destiné à assurer une liaison rigide entre deux tiges (1, 2) d'un système d'ostéosynthèse rachidienne, constitué par un crochet (4) adapté pour pouvoir coiffer de manière coulissante une barre transversale rigide (3), caractérisé en ce que ce crochet comporte un corps (5) et deux lames (6) distantes d'un intervalle (d) de largeur correspondant à celle de la barre (3), et une portée d'appui (7) du crochet sur la barre est ménagée sur le corps (5) entre les lames (6), lesquelles s'étendent de chaque côté de la barre lorsque le crochet chevauche cette dernière, ce crochet étant équipé de moyens (9, 8) de blocage sur ladite barre.

2. Elément selon la revendication 1, caractérisé en ce que la portée (7) est plane afin de pouvoir prendre appui sur une face plane (3a) d'une barre (3) de section rectangulaire, les deux lames (6) s'étendant parallèlement l'une à l'autre de part et d'autre de ladite portée.

3. Elément selon l'une des revendications 1 et 2, caractérisé en ce que les deux lames (6) sont recourbées suivant un rayon de courbure correspondant à celui des tiges (1, 2), et ainsi adaptées pour envelopper celles-ci, ces lames présentant des surfaces incurvées (6a) d'appui des tiges.

4. Elément selon l'une des revendications 1 à 3, caractérisé en ce que les moyens de blocage comportent un bouchon fileté (9) et un trou taraudé correspondant (8), agencé dans le corps (5), ce trou débouchant sur la portée d'appui (7) de la barre (3) afin de permettre le blocage de cette dernière sur la tige (1; 2) par le bouchon (9) vissé dans le trou (8).

5. Dispositif de fixation transverse pour assurer une liaison transverse rigide entre deux tiges (1, 2) d'un système d'ostéosynthèse rachidienne, caractérisé en ce qu'il comprend en combinaison :
- une barre rigide (3) de longueur appropriée,
- et une paire d'éléments de fixation (4) de la barre sur les tiges, conformes à l'une des revendications 1 à 4, pouvant être fixés de manière réglable en une position choisie sur la barre (3).

6. Dispositif selon la revendication 5, caractérisé en ce que la barre (3) a une section rectangulaire et la portée (7) de chaque élément de fixation (4) est plane afin de permettre l'appui de cet élément sur l'une des faces de la barre.

7. Dispositif selon la revendication 6, caractérisé en ce qu'un orifice (11) profilé pour recevoir un outil de vissage est agencé dans le bouchon (9).

## Claims

1. Fixing element (4) for a transverse fixing device designed to guarantee a rigid link between two rods (1, 2) in a rachidian osteosynthesis system, comprising a hook (4) adapted to be able to seize a rigid transverse bar (3) in a sliding manner, characterised by the fact that this hook comprises a body (5) and two blades (6) distanced by an interval (d) in width which corresponds to that of the bar (3), and a support bearing (7) for the hook on the bar is located on the body (5) between the blades (6), which extends on each side of the bar when the hook crosses the latter, the hook being fitted with blocking means (9, 8) on the side bar.

2. Element according to claim 1, characterised in that the bearing (7) is flat to be able to support a flat face (3a) of a bar (3) with a rectangular section, the two blades (6) extending in parallel on one side and the other of the said bearing.

3. Element according to one of claims 1 and 2, characterised in the fact that these two blades (6) are curved according to a curvature radius which corresponds to that of the rods (1, 2) and thus adapted to encircle them, these blades having non-curved support surfaces (6a) for the rods.

4. Element according to one of claims 1 to 3, characterised in that the blocking means comprise a threaded stopper (9) and a corresponding screw cut hole (8), fitted in the body (5), this hole opening out onto the support bearing (7) of the bar (3) in order to allow the latter to be blocked on the rod (1, 2) by the stopper (9) screwed into the hole (8).

5. Transverse fixing device to guarantee a rigid transverse rigid connection between two rods (1, 2) of a rachidian osteosynthesis connection, characterised in that it comprises in combination:-
· a rigid bar (3) of an appropriate length,
· and a pair of bar fixing elements (4) on the rods, in accordance with one of claims 1 to 4, which can be fixed in an adjustable manner in a chosen position on the bar (3).

6. Device according to claim 5, characterised in that the bar (3) has a rectangular section and the bearing (7) of each fixing element (4) is flat to allow this element to be supported on one of the faces of the bar.

7. Device according to claim 6, characterised in that a profiled hole (11) to take the screwing tool is fitted in the stopper (9).

## Patentansprüche

1. Befestigungselement (4) für eine transversale Befestigungsvorrichtung zur Sicherung einer starren Verbindung zwischen zwei Stäben (1, 2) eines Wirbelsäulenstützsystems, das aus einem Haken (4) besteht, welcher auf verschiebbare Weise eine starre Querstange (3) umfassen kann, dadurch gekennzeichnet, daß der Haken einen Hauptteil (5) und zwei Arme (6) umfaßt, die in einem Abstand (d) voneinander angeordnet sind, dessen Größe der Größe der Stange (3) entspricht, und daß eine Auflagefläche (7) des Hakens auf der Stange am Hauptteil (5) zwischen den Armen (6) vorgesehen ist, wobei sich die Arme von jeder Seite der Stange aus erstrecken, wenn der Haken diese übergreift, und wobei der Haken mit Blockiereinrichtungen (9, 8) an der Stange versehen ist.

2. Element nach Anspruch 1, dadurch gekennzeichnet, daß die Auflagefläche (7) eben ausgebildet ist, um eine Lagerung auf einer ebenen Fläche (3a) einer Stange (3) mit rechteckigem Querschnitt zu ermöglichen, und daß sich die beiden Arme (6) parallel zueinander beiderseits der Auflagefläche erstrecken.

3. Element nach einem der Ansprüche 1 und 2, dadurch gekennzeichnet, daß die beiden Arme (6) mit einem Krümmungsradius umgebogen sind, der dem der Stäbe (1, 2) entspricht, und somit in der Lage sind, die Stäbe zu umhüllen, und daß die Arme einwärts gekrümmte Lagerflächen (6a) für die Stäbe bilden.

4. Element nach einem der Ansprüche 1 bis 3, dadurch gekennzeichnet, daß die Blockiereinrichtungen einen Schraubbolzen (9) und eine entsprechende Bohrung (8) mit Innengewinde, die im Hauptteil (5) angeordnet ist, umfassen, wobei die Bohrung an der Auflagefläche (7) der Stange (3) mündet, um eine Blockierung derselben am Stab (1; 2) über den in die Bohrung (8) eingeschraubten Schraubbolzen (9) zu ermöglichen.

5. Transversale Befestigungsvorrichtung zur Sicherung einer starren transversalen Verbindung zwischen zwei Stäben (1, 2) eines Wirbelsäulenstützsystems, dadurch gekennzeichnet, daß sie in Kombination umfaßt:
- eine starre Stange (3) mit geeigneter Länge und
- ein Paar von Befestigungselementen (4) der Stange an den Stäben gemäß einem der Patentansprüche 1 bis 4, die verstellbar in einer ausgewählten Position an der Stange (3) fixiert werden können.

6. Vorrichtung nach Anspruch 5, dadurch gekennzeichnet, daß die Stange (3) einen Rechteckquerschnitt besitzt und daß die Auflagefläche (7) eines jeden Befestigungselementes (4) eben ist, um eine Lagerung des Elementes auf einer der Seiten der Stange zu ermöglichen.

7. Vorrichtung nach Anspruch 6, dadurch gekennzeichnet, daß im Schraubbolzen (9) eine profilierte Öffnung (11) zur Aufnahme eines Schraubwerkzeuges angeordnet ist.
